# EUROPEAN PATENT APPLICATION

(11) **EP 1 110 959 A1**
(43) Date of publication of application: **27.06.2001**
(21) Application number: 00942379.9
(22) Date of filing: 29.06.2000
(51) Int. Cl.: C07D 307/30, A61K 31/366

(54) **PROCESS FOR SELECTIVE LACTONIZATION**

(30) Priority: 29.06.1999 JP 18364099
(71) Applicant: KANEKA CORPORATION, Osaka-shi, Osaka 530-8288 (JP)
(72) Inventor: FUKAE, Masafumi, Takasago-shi, Hyogo 676-0026 (JP); UEDA, Makoto, Akashi-shi, Hyogo 673-0018 (JP); TATSUKI, Kenichi, Kakogawa-shi, Hyogo 675-0022 (JP)
(74) Representative: Ulmann, Catherine Claire
(86) International application number: JP0004269
(87) International publication number: WO0100606

(57) **Abstract**

The invention has its object to provide a method of lactonization for a compound of the general formula (1) in which the dimer formation reaction can be drastically inhibited.

The invention provides a process for producing a compound of the general formula (2)
comprising lactonizing a compound of the general formula (1) under conditions such that the solubility of the compound of the general formula (1) and/or the compound of the general formula (2) is not more than 0.5 w/w % to thereby give a compound of the general formula (2) with a dimer content of not more than 0.3 mole %.

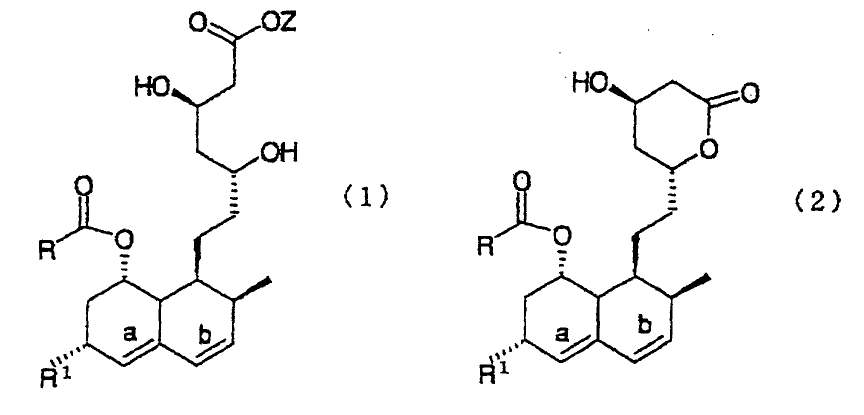

## Description

### TECHNICAL FIELD

The present invention relates to a production process starting with a free acid of the following general formula (1) to give a lactone compound of the following general formula (2).

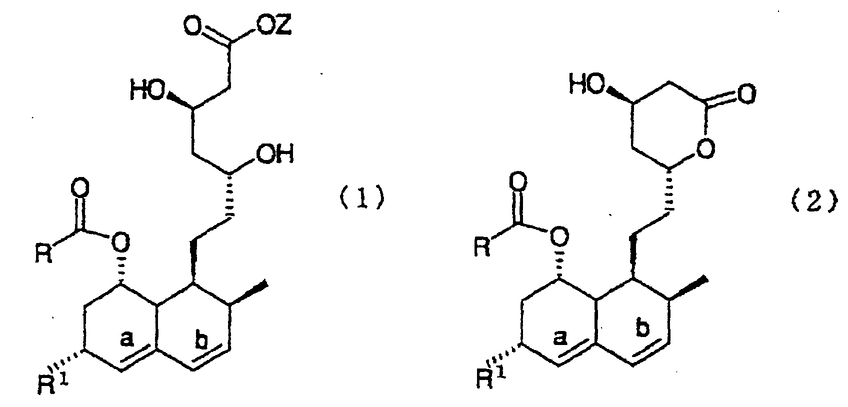

### BACKGROUND ART

Among the antihyperlipemic drugs commercially available today, a group of highly functional drugs capable of restricting cholesterol biosynthesis through inhibition of the enzyme HMG-CoA reductase have the following chemical formula (4) or (5). 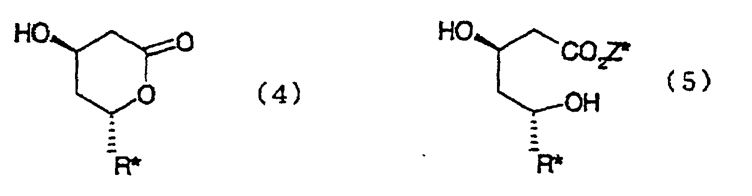 [wherein Z* represents hydrogen, a C₁₋₅ alkyl group, or a C₁₋₅ alkyl group substituted with one member selected from the group consisting of phenyl, dimethylamino and acetylamino; R* represents a group of the following general formula (6): 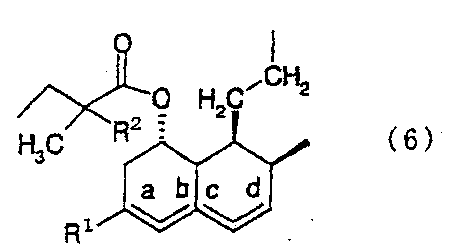 (wherein R¹ represents CH₃ or OH, R² represents H or CH₃, a, b, c and d are optional double bonds, particularly b and d are double bonds or all of a, b, c and d are single bonds) ] . Many of the native compounds and their semisynthetic analogs are commercially available in the lactone form and, therefore, it is of some great importance to use a high-yield, efficient technology for the lactonization of free acids or salts.

Heretofore, lactonization of these compounds has been achieved by heating the starting compound in an organic solvent in which it is soluble to a sufficient extent, such as toluene, ethyl acetate and isopropyl acetate, under refluxing or substantially refluxing conditions (EP 0033536 A2). Lactonization is catalyzed by an acid. The necessary acidity is provided by the substrate carboxylic acid itself or addition of a strong acid, whereby the lactonization is rendered feasible at a comparatively low temperature.

Lactonization is an equilibrium reaction and in order to obtain a lactonization product in high yield, the equilibrium must be shifted toward the lactone side by suitable means. Usually, a lactonization reaction can be substantially carried through by using an azeotropic distillation technique and/or passing nitrogen to remove reaction byproducts (e.g. water and ammonia) from the reaction system.

There is known a process in which the necessary shift of equilibrium in a lactonization reaction is attained in a different way. Here, the equilibrium between the lactone and the starting free acid is shifted toward the lactone side by carrying out the reaction and ripening in acetic acid/trifluoroacetic acid at low temperature and, then, adding a suitable amount of water to precipitate the product lactone (JP H09-188672 A1).

The conventional lactonization methods have several drawbacks. For example, while the substrate free acid acts as an acidic catalyst by itself, the reaction time is protracted as the substrate is consumed progressively, with the result that the formation of byproducts is encouraged and the reaction velocity declines. As the product lactone is exposed to the remaining free acid for an extended time under such conditions, the 3-hydroxyl group of the lactone undergoes esterification reaction with the free acid so that the dimer of the following formula (3) is increasingly produced. Since this dimer can hardly be removed by a recrystallization technique (JP H09-188672 A1), the purity and yield of the product are lowered.

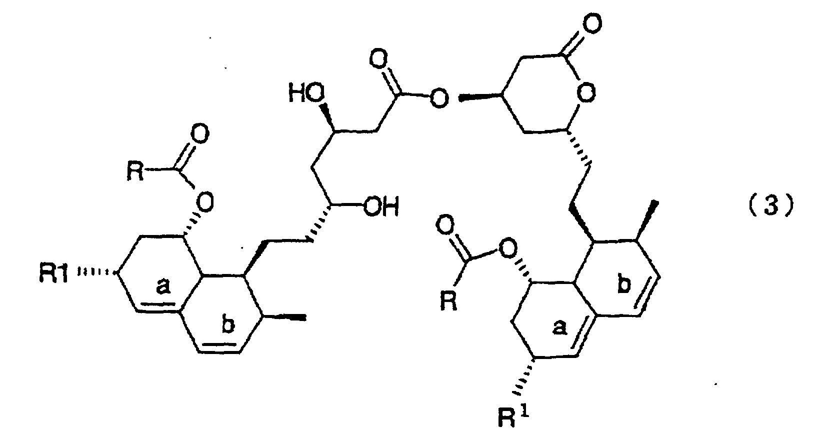

A method for minimizing the formation of the above dimer may comprise performing the lactonization reaction under high dilution conditions. However, it is obvious that such a practice would detract from productivity.

In the case of the above-mentioned reaction in acetic acid/trifluoroacetic acid according to JP H09-188672 A1 referred to above, the dimer amount is reportedly reduced to not higher than 0.2% but this method is unsuitable for practical use, for the reaction at low temperature needs long reaction time, water must be repeatedly added for shifting the equilibrium, and solvent recovery is essential.

### DISCLOSURE OF INVENTION

The inventors of the present invention made an intensive investigation for overcoming the above disadvantages and, guided by the notion that lactonization is a self-condensation reaction, discovered that by conducting a lactonization reaction in a medium in which the substrate free acid (1) and/or the product lactone (2) is substantially insoluble, the dimer formation reaction between product lactone (2) and free acid (1) can be drastically inhibited, thus enabling establishment of quite a novel lactonization technology. The present invention has accordingly been developed.

Thus, the present invention is concerned with a process for producing a compound of the above general formula (2) through lactonization of a compound of the above general formula (1)
which comprises carrying out the lactonization reaction under a condition such that the solubility of the compound of the general formula (1) and/or that of the compound of the general formula (2) are/is not more than 0.5 w/w % to thereby give a compound of the general formula (2) with a dimer content of not more than 0.3 mole %, said dimer being of the above general formula (3).

The present invention is now described in detail.

Referring to the above formulas (1), (2) and (3), R represents a C₁₋₁₀ alkyl group, R¹ represents CH₃, CH₂OH, CH₂OCOR², CO₂R³, CONR⁴(R⁵), OH, CH₂OR² or CH₂NR⁴(R⁵), Z represents H, NH₄⁺ or a metal cation, R² represents a C₁₋₅ alkyl group, R³ represents H or a C₁₋₅ alkyl group, R⁴ and R⁵ are each independently selected from hydrogen and C₁₋₅ alkyl groups, and a and b are simultaneously double bonds, either a or b is a single bond, or a and b are single bonds.

R is preferably a sec-butyl group or a 1,1-dimethylpropyl group. R¹ is preferably CH₃ or OH, particularly CH₃. Z is preferably H or NH₄⁺, particularly H. It is preferable that both a and b is double bonds.

It is particularly preferable that R¹ is CH₃ or OH and Z is H or NH₄⁺. It is also preferable that R is a sec-butyl group or a 1,1-dimethylpropyl group, R¹ is CH₃ or OH, and both a and b are double bonds. It is particularly preferable that R is a sec-butyl group or a 1,1-dimethylpropyl group, R¹ is CH₃, Z is H, and both a and b are double bonds.

The free acid (1) to be used as the substrate in the present invention can be obtained as a culture product by the technologies described in, inter alia, WO 94/10328 and WO 98/50572.

The free acid (1) for use as the substrate in the present invention may generally be the free acid (1) and/or a partially cyclized composition comprised of the free acid (1) and lactone (2), which can be obtained from a culture broth by a suitable means such as extraction. The suitable means mentioned above includes a method comprising alkaline extraction and recovery from a cell-free extract as described in WO 94/10328 and a isolation method using free acids in which an alkaline cell-free extract is further purified by an adsorption treatment with a resin as described in WO 98/50572. It is also possible to use a solution prepared by substitution of a lactonization reaction medium for solvents after extract.

The lactonization reaction according to the present invention is carried out under conditions such that the solubility of the above compound of the general formula (1) and/or that of the above compound of the general formula (2) will not exceed 0.5 w/w %. Thus, in the present invention, the conditions of lactonization reaction are so controlled that the solubility of the compound of the general formula (1) only or the solubility of the compound of the general formula (2) only, or both the solubility of the compound of the general formula (1) and that of the compound of the general formula (2) will be 0.5 w/w % or less. To insure such solubility, it is not only necessary to select a suitable reaction medium but also judiciously adjust the reaction temperature, reaction pressure and the acidity. The term solubility in this context means the weight percent based on the whole weight of reaction mixture.

The reaction medium for the lactonization reaction according to the present invention may be any medium in which the solubility of free acid (1) and/or that of lactone (2) under the lactonization conditions will be not over 0.5 w/w % and, to mention an additional requirement, it is preferably a medium which is stable under the lactonization conditions. The medium includes C₅₋₁₆ saturated hydrocarbon media represented by the formula CₙH₂ₙ₊₂ or CₙH₂ₙ. Among such media, either one or a plurality of them can be used. Preferred saturated hydrocarbon medium includes pentane, 2-methylpentane, 2, 2-dimethylpentane, hexane, iso-hexane, normal heptane, octane, iso-octane, methylcyclopentane, ethylcyclopentane, propylcyclopentane, methylcyclohexane, ethylcyclohexane, propylcyclohexane and mixtures thereof.

Provided, of course, that it is stable under lactonization conditions, a C₅₋₁₆ unsaturated hydrocarbon medium or media represented by CₙH₂ₙ or CₙH₂ₙ₋₂ can also be used and mixtures of said saturated hydrocarbon medium or media with said unsaturated hydrocarbon medium or media may also be employed. As far as the condition that the solubility of free acid (1) and/or that of lactone (2) therein are/is not more than 0.5 w/w % and the condition that the medium is stable are both satisfied under the lactonization conditions, an aqueous medium may also be used and a mixture of said aqueous medium or media with said hydrocarbon medium or media can also be employed.

In a preferred mode of practice of the present invention, the medium for lactonization reaction is a mixture of water and at least one member selected from the group consisting of C₅₋₁₆ saturated hydrocarbon media of the formula CₙH₂ₙ₊₂ or CₙH₂ₙ and C₅₋₁₆ unsaturated hydrocarbon media of the formula CₙH₂ₙ or CₙH₂ₙ₋₂.

In another preferred mode, water is used as the lactonization reaction medium.

The amount of use of the medium may usually be about 80 to 98 w/w % based on the total weight of free acid (1) and medium.

This reaction is effectively promoted in the presence of an acid; for example, when the acidity is expressed in pH-value, the reaction is generally carried out under acidic conditions not over pH 5. The acid necessary for establishing an acidity of not over pH 5 includes formic acid, phosphoric acid, trifluoroacetic acid, sulfuric acid, hydrochloric acid, perchloric acid, p-toluenesulfonic acid and methanesulfonic acid, among others. These acids may be used alone or a plurality of them may be used in a suitable combination. For practical purposes, the reaction is promoted by adjusting the pH of the system to not over 5 with a mineral acid which is comparatively inexpensive but the addition of plural acids is acceptable, for example, it is acceptable to bring the pH down to a certain level with a mineral acid and, then, add an organic acid. With the progress of lactonization, the reaction velocity drops but it has been demonstrated that the necessary promotion of reaction can be attained by supplemental addition of said acid in the course of reaction.

The lactonization reaction temperature may be as low as, for example, about 5 °C but from productivity points of view, the reaction temperature is preferably not below 20 °C, and in consideration of the stability of the medium and substrate, the temperature range of 40 to 80 °C is still more preferred.

When water forms as a byproduct, it is good practice to use water and a hydrocarbon medium in combination as the reaction medium and conduct lactonization while the water is azeotropically distilled off. In this case, removal of water is preferably accompanied by simultaneous addition of the hydrocarbon medium.

The lactonization reaction may be carried out at atmospheric pressure but it may optionally be conducted under a reduced pressure of about 30 to 600 mmHg while the medium is refluxed at a comparatively low temperature.

The time necessary for this lactonization is usually about 1 to 30 hours.

Isolation of the product lactone (2) is generally very easy. Thus, since the objective lactone (2) has a very low solubility in the reaction medium, it can be easily isolated by subjecting the reaction mixture to solid-liquid separation either at a stage of reaction or on completion of reaction or, depending on the medium used, by cooling the reaction mixture and then subjecting it to solid-liquid separation. Where necessary, it is of course possible to increase the purity of the product by a purification procedure such as recrystallization.

By the production method of the present invention, a lactone (2) with a byproduct dimer (3) content of not more than 0.3 mole % can be obtained. In a preferred mode of practice, even a lactone (2) with a byproduct dimer (3) content of as low as 0.2 mole % or less can be obtained.

### BEST MODE FOR CARRYING OUT THE INVENTION

The following examples illustrate the present invention in further detail, it being, of course, to be understood that the scope of the invention is by no means restricted to these specific examples.

In the following examples, the free acid and/or the lactone was assayed by high performance liquid chromatography under the following conditions (Analytical Conditions A).

### Analytical Conditions A

| | |
|---|---|
| Instrument | Shimadzu Corporation LC-10A |
| Column | Nacalai Tesque ODS column, Cosmosil |
| | 5C18-AR-300 |
| Eluent | acetonitrile-0.1% phosphoric acid/H₂O |
| | = 48:52 (v/v) |
| Flow rate | 2.0 ml/min |
| Detection | 238 nm (UV detector) |
| Temperature | 45 °C |

The dimer formed as a lactonization reaction byproduct was assayed by high performance liquid chromatography under the following conditions (Analytical Condition B).

### Analytical Conditions B

| | |
|---|---|
| Instrument | Shimadzu Corporation LC-10A |
| Column | Nacalai Tesque ODS column, Cosmosil |
| | 5C18-AR-300 |
| Eluent | acetonitrile-0.1% phosphoric acid/H₂O |
| | = 80:20 (v/v) |
| Flow rate | 1.0 ml/min |
| Detection | 238 nm (UV detector) |
| Temperature | 45 °C |

### [Example 1]

### Production of 6(R)-[2-[8(S)-(2-methylbutyryloxy)-2-(S),6(R)-dimethyl-1,2,6,7,8,8a(R)-hexahydronaphthyl-1(S)]ethyl]-4(R)-hydroxy-3,4,5,6-tetrahydro-2H-pyran-2-one (in the general formula (1), R = sec-butyl, R¹ = CH₃, a and b = double bonds, Z = H).

An aqueous solution (85 ml) of 7-1,2,6,7,8,8a(R)-hexahydro-2(S),6(R)-dimethyl-8(S)-(2-methylbutyryloxy)-1(S)-naphthyl-3(R),5(R)-dihydroxyheptanoic acid (the concentration of the substrate heptanoic acid = 4.0 weight %) was adjusted to pH 3 with sulfuric acid and heated to 70 °C with stirring. After 5 hours of reaction, a portion of the slurry was sampled and analyzed by HPLC to confirm that the lactonization rate was at least 70%. The heating was terminated after 6 hours of reaction, and after cooling to room temperature, the slurry-like solid matter was recovered by suction filtration, dried in vacuo at 50 °C, and analyzed by HPLC to confirm that the lactone had been produced in 78% yield. The dimer content of the product lactone was found to be as low as 0.11 mole %. Incidentally, the solubility of the substrate heptanoic acid and that of the product lactone.were both not more than 0.05 w/w %.

### [Example 2]

### Production of 6(R)-[2-[8(S)-(2,2-dimethylbutyryloxy)-2(S),6(R)-dimethyl-1,2,6,7,8,8a(R)-hexahydronaphthyl-1(S)]ethyl]-4(R)-hydroxy-3,4,5,6-tetrahydro-2H-pyran-2-one (in the general formula (1), R = 1, 1-dimethylpropyl, R¹ = CH₃, a and b = double bonds, Z = H).

An aqueous solution (100 ml) of 7-1,2,6,7,8,8a(R)-hexahydro-2(S),6(R)-dimethyl-8(S)-(2,2-dimethylbutyryloxy)-1(S)-naphthyl-3(R),5(R)-dihydroxyheptanoic acid (the concentration of the substrate heptanoic acid = 3.6 weight %) was adjusted to pH 3 with sulfuric acid and heated to 70 °C with stirring. After 5 hours of reaction, a portion of the slurry was sampled and analyzed by HPLC to confirm that the lactonization rate was at least 70%. The heating was terminated after 6 hours of reaction, and after cooling to room temperature, the slurry-like solid matter was recovered by suction filtration, dried in vacuo at 50 °C, and analyzed by HPLC to confirm that the lactone had been produced in 76% yield. The dimer content of the product lactone was found to be as low'as 0.13 mole %. Incidentally, the solubility of the substrate heptanoic acid and that of the product lactone were both not more than 0.05 w/w %.

### [Example 3]

### Production of 6(R)-[2-[8(S)-(2-methylbutyryloxy)-2(S),6(R)-dimethyl-1,2,6,7,8,8a(R)-hexahydronaphthyl-1(S)]ethyl]-4(R)-hydroxy-3,4,5,6-tetrahydro-2H-pyran-2-one (in the general formula (1), R = sec-butyl, R¹ = CH₃, a and b = double bonds, Z = H).

An aqueous solution (85 ml) of 7-1,2,6,7,8,8a(R)-hexahydro-2 (S),6(R)-dimethyl-8(S)-(2-methylbutyryloxy)-1(S)-naphthyl-3(R),5(R)-dihydroxyheptanoic acid (the concentration of the substrate heptanoic acid = 4.0 weight %) was adjusted to pH 3 with sulfuric acid, followed by addition of 80 ml of normal heptane. In a flask equipped with a Dean-Stark trap, the above mixture was refluxed by heating at an internal temperature of 60 °C under reduced pressure (ca 220 mmHg). In the course of reaction, the trapped water was removed and the corresponding amount of n-heptane was added. The refluxing under reduced pressure was continued until the azeotropic distillation of water had substantially ceased and after an additional 4 hours for ripening, a portion of the slurry was sampled and analyzed by HPLC to confirm that the lactonization had progressed to not less than 90%. Therefore, the heating was discontinued and the reaction mixture was cooled to room temperature. The slurry-like solid was recovered by suction filtration, dried in vacuo at 50 °C, and analyzed by HPLC to confirm that the lactone had been produced in 95% yield. The dimer content of the lactone was found to be as low as 0.11 mole %. Incidentally, the solubility of the substrate heptanoic acid and that of the product lactone were not more than 0.05 w/w % and not more than 0.08 w/w %, respectively, throughout the entire reaction time.

### [Example 4]

### Production of 6(R)-[2-[8(S)-(2,2-dimethylbutyryloxy)-2(S),6(R)-dimethyl-1,2,6,7,8,8a(R)-hexahydronaphthyl-1(S)]ethyl]-4(R)hydroxy-3,4,5,6-tetrahydro-2H-pyran-2-one (in the general formula (1), R = 1,1-dimethylpropyl, R¹ = CH₃, a and b = double bonds, Z = H).

An aqueous solution (100 ml) of 7-1,2,6,7,8,8a(R)-hexahydro-2(S),6(R)-dimethyl-8(S)-(2,2-dimethylbutyryloxy)-1(S)-naphthyl-3(R),5(R)-dihydroxyheptanoic acid (the concentration of the substrate heptanoic acid = 3.6 weight %) was adjusted to pH 3 with sulfuric acid, followed by addition of 80 ml of normal heptane. In a flask equipped with a Dean-Stark trap, the above mixture was refluxed by heating at an internal temperature of 40 °C under reduced pressure (ca 100 mmHg). The water was azeotropically removed by the same procedure as in Example 3 until the distillation of water had substantially ceased. After an additional 4 hours for ripening, a portion of the slurry was sampled and analyzed by HPLC to confirm that the lactonization had progressed to not less than 90%. Therefore, the heating was discontinued and the reaction mixture was cooled to room temperature. The slurry-like solid was recovered by suction filtration, dried in vacuo at 50 °C, and analyzed by HPLC to confirm that the lactone had been produced in 94% yield. The dimer content of the product lactone 'was found to be as low as 0.26 mole %. Incidentally, the solubility of the substrate heptanoic acid and that of the product lactone were not more than 0.07 w/w % and not more than 0.21 w/w %, respectively, throughout the entire reaction time.

### [Example 5]

Except that a 10% aqueous solution of sulfuric acid, 10 mole % based on the substrate, was further added before completion of the azeotropic distillation of water so as to promote lactonization, the lactonization reaction was conducted under otherwise the same substrate and reaction conditions as in Example 3. The supplemental addition of the acid in the course of reaction promoted lactonization so that the ripening time following azeotropic distillation was curtailed by 1 hour and, in addition, the lactonization rate was increased. After 3 hours of ripening, the refluxing was discontinued and the reaction mixture was cooled to room temperature. The solid matter was recovered by suction filtration, dried in vacuo at 50 °C, and analyzed by HPLC to confirm that the lactone had been formed in 97% yield. The dimer content of the product lactone was found to be as low as 0.12 mole % . The solubility of the substrate heptanoic acid and that of the product lactone were not more than 0.05 w/w % and not more than 0.08 w/w %, respectively, throughout the entire reaction time.

### [Example 6]

Except that 10 mole % of trifluoroacetic acid, based on the substrate, was added immediately on substantial completion of azeotropic distillation of water for the purpose of promoting lactonization, the lactonization reaction was conducted under the same substrate and reaction conditions as in Example 3. Just as in Example 5, supplemental addition of an acid promoted lactonization so that conversion rate was increased. Thus, after the same treatment as in Example 5, the product was analyzed by HPLC. The analysis revealed formation of the, lactone in 97% yield. The dimer content of the product lactone was found to be as low as 0.12 mole %. The solubility of the substrate heptanoic acid and that of the product lactone were not more than 0.06 w/w % and not more than 0.08 w/w %, respectively, throughout the entire reaction time.

### [Example 7]

Except that hexane was used in lieu of normal heptane, the lactonization reaction was conducted under the same substrate and reaction conditions as in Example 3. After the same treatment as in Example 3 (however, the reduced pressure was set at 550 to 600 mmHg), it was confirmed by HPLC analysis that the lactone was formed in 95% yield. The dimer content of the product lactone was found to be as low as 0.09 mole %. The solubility of the substrate heptanoic acid and that of the product lactone were not more than 0.05 w/w % and not more than 0.06 w/w %, respectively, throughout the entire reaction time.

### [Example 8]

Except that normal octane was used in lieu of normal heptane, the lactonization reaction was carried out under the same substrate and reaction conditions as in Example 3. After the same treatment as in Example 3 (however, the reduced pressure was 70 to 90 mmHg), it was confirmed by HPLC analysis that the lactone was formed in 94% yield. The dimer content of the product lactone was found to be as low as 0.10 mole %. The solubility of the substrate heptanoic acid and that of the product lactone were not more than 0.05 w/w % and not more than 0.08 w/w %, respectively, throughout the entire reaction time.

### [Example 9]

Except that methylcyclohexane was used in lieu of normal heptane and the reaction temperature was altered to 70 °C, the lactonization reaction was carried out under the same substrate and pH conditions as in Example 3. After the same treatment as in Example 3, it was confirmed by HPLC analysis that the lactone was formed in 97% yield. The dimer content of the product lactone was found to be as low as 0.21 mole %. The solubility of the substrate heptanoic acid and that of the product lactone were not more than 0.05 w/w % and not more than 0.11 w/w %, respectively, throughout the entire reaction time.

### [Example 10]

Except that the pH adjustment with sulfuric acid prior to the start of reaction was made to pH 2, the lactonization reaction was conducted under the same substrate and temperature conditions as in Example 3. After the same treatment as in Example 3, it was confirmed by HPLC analysis that the lactone was formed in 95% yield. The dimer content of the product lactone was found to be as low as 0.12 mole %. The solubility of the substrate heptanoic acid and that of the product lactone were not more than 0.05 w/w % and not more than 0.08 w/w %, respectively, throughout the entire reaction time.

### [Example 11]

Except that the pH adjustment with sulfuric acid prior to the start of reaction was made to pH 5 and the reaction temperature was altered to 80 °C, the lactonization reaction was conducted for 30 hours under the same substrate and medium conditions as in Example 1. After the same treatment as in Example 1, it was confirmed by HPLC analysis that the lactone was formed in 81% yield. The dimer content of the product lactone was found to be as low as 0.27 mole %. The solubility of the substrate heptanoic acid and that of the product lactone were not more than 0.08 w/w % and not more than 0.10 w/w %, respectively, throughout the entire reaction time.

### [Comparative Example 1]

Except that toluene was used in lieu of normal heptane, the lactonization reaction was carried out under the same substrate and reaction conditions as in Example 3. After the same treatment as in Example 3, HPLC analysis of the solid product revealed that the yield of the lactone was 40%. However, when the amount of the lactone in the filtrate was added to the above figure, the rate of conversion to the lactone was 98%. However, the dimer content of the product lactone was as high as 0.63 mole %. When the solubility of the lactone under the reaction conditions was separately checked by HPLC analysis, it was found to be not less than 2%.

### INDUSTRIAL APPLICABILITY

In accordance with the present invention wherein a free acid of the general formula (1) is lactonized in a reaction medium in which the solubility of said free acid (1) and/or that of the product lactone (2) are/is not greater than 0.5 w/w %, the formation of the dimer of the general formula (3) due to a side reaction between the free acid (1) and lactone (2), which dimer cannot be easily removed by the conventional purification procedure, e.g. recrystallization, is drastically diminished. Thus, the present invention provides a very effective technology for the production of lactones of the general formula (2).

## Claims

1. A process for producing a compound of the general formula (2): 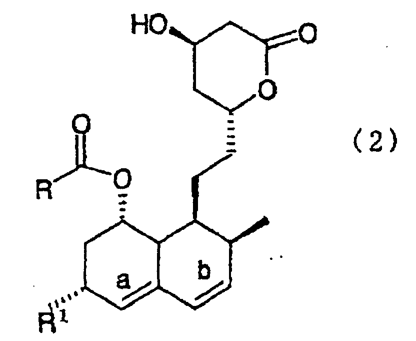 in the formula, R represents a C₁₋₁₀ alkyl group, R¹ represents CH₃, CH₂OH, CH₂OCOR², CO₂R³, CONR⁴(R⁵), OH, CH₂OR² or CH₂NR⁴(R⁵), R² represents a C₁₋₅ alkyl group, R³ represents H or a C₁₋₅ alkyl group, R⁴ and R⁵ each is independently selected from among hydrogen and C₁₋₅ alkyl groups, and both a and b are double bonds, one of a and b is a single bond with the other being a double bond, or both a and b are single bonds,
comprising lactonizing a compound of the general formula (1): 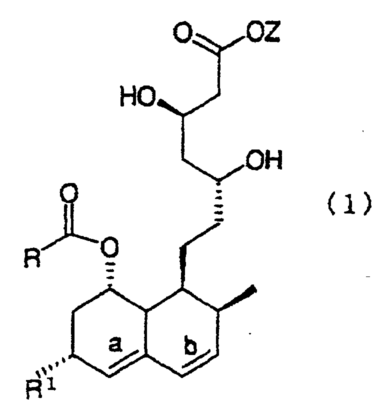 in the formula, R, R¹, a and b are as defined above, Z represents H, NH₄⁺ or a metal cation,
wherein the lactonization reaction is carried out under a condition such that the solubility of said compound of the general formula (1) and/or that of said compound of the general formula (2) are/is not greater than 0.5 w/w % to thereby produce said compound of the general formula (2) with a dimer content of not more than 0.3 mole %,
and said dimer is represented by the general formula (3) : 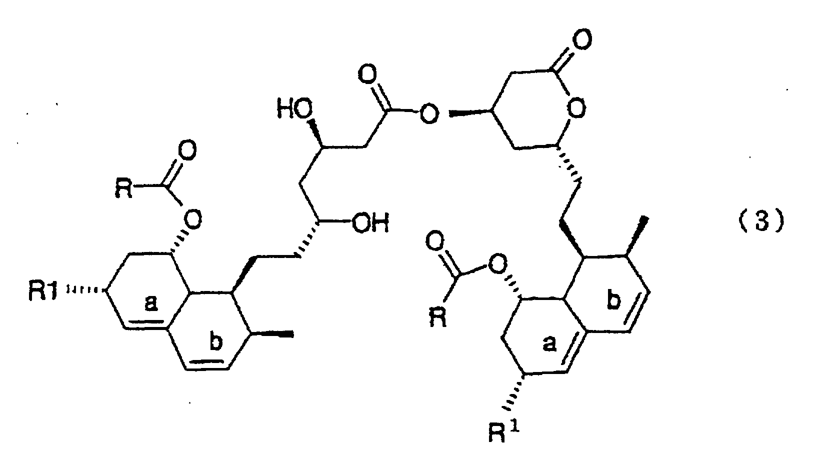 in the formula, R, R¹, a and b are as defined above.

2. The process according to Claim 1
wherein R¹ is CH₃ or OH and Z is H or NH₄⁺.

3. The process according to Claim 1 or 2
wherein a medium for the lactonization reaction comprises at least one member selected from water, C₅₋₁₆ saturated hydrocarbons of the formula CₙH₂ₙ₊₂ or CₙH₂ₙ and C₅₋₁₆ unsaturated hydrocarbons of the formula CₙH₂ₙ or CₙH₂ₙ₋₂.

4. The process according to Claim 3
wherein the medium for the lactonization reaction comprises water and at least one member selected from C₅₋₁₆ saturated hydrocarbons of the formula CₙH₂ₙ₊₂ or CₙH₂ₙ and C₅₋₁₆ unsaturated hydrocarbons of the formula CₙH₂ₙ or CₙH₂ₙ₋₂.

5. The process according to Claim 3
wherein the medium for the lactonization reaction comprises water.

6. The process according to Claim 3 or 4
wherein the hydrocarbon medium for the lactonization reaction comprises at least one member selected from the group consisting of pentane, 2-methylpentane, 2,2-dimethylpentane, hexane, iso-hexane, normal heptane, octane, iso-octane, methylcyclopentane, ethylcyclopentane, propylcyclopentane, methylcyclohexane, ethylcyclohexane and propylcyclohexane.

7. The process according to any of Claims 1 to 6
wherein the lactonization reaction is carried out under an acidic condition not over pH 5.

8. The process according to Claim 7
wherein the acidic condition not over pH 5 is established by adding at least one acid selected from the group consisting of formic acid, phosphoric acid, trifluoroacetic acid, sulfuric acid, hydrochloric acid, perchloric acid, p-toluenesulfonic acid and methanesulfonic acid.

9. The process according to any of Claims 1 to 8
wherein R is a sec-butyl group or a 1,1-dimethylpropyl group, R¹ is CH₃ or OH, and a and b are double bonds.

10. The process according to Claim 9
wherein R is a sec-butyl group or a 1,1-dimethylpropyl group, R¹ is CH₃, Z is H, and a and b are double bonds.
